# EUROPEAN PATENT SPECIFICATION

(11) **EP 0 717 727 B1**
(45) Date of publication and mention of the grant of the patent: **18.11.1998**
(21) Application number: 94909183.9
(22) Date of filing: 11.03.1994
(51) Int. Cl.: C07C 17/00, C07C 19/08, B01J 23/60

(54) **CATALYTIC METHOD OF REPLACING HALOGEN IN HALOCARBONS**
KATALYTISCHES VERFAHREN ZUM AUSTAUSCH VON HALOGEN IN HALOGENKOHLENWASSERSTOFFEN
PROCEDE CATALYTIQUE DE REMPLACEMENT DES HALOGENES DANS LES HALOCARBONES

(30) Priority: 26.03.1993 GB 9306334
(43) Date of publication of application: 26.06.1996
(73) Proprietor: THE UNIVERSITY COURT OF THE UNIVERSITY OF DUNDEE, Dundee DD1 4HN, Scotland (GB)
(72) Inventor: THOMSON, James, Stobswell, Dundee DD4 7DA (GB)
(74) Representative: Pattullo, Norman
(86) International application number: GB9400477
(87) International publication number: WO9422792

(56) References cited:
- EP-A- 0 347 830
- EP-A- 0 508 660
- WO-A-92/18447
- WO-A-93/09080
- DATABASE WPI Section Ch, Week 8933, Derwent Publications Ltd., London, GB; Class E16, AN 89-237716 & JP,A,1 172 349 (ASAHI GLASS KK) 7 July 1989

## Description

This invention relates to a catalytic method of replacing halogen in halo-substituted hydrocarbons by hydrogen, and especially when only a selected member of the halogen family is replaced. Thus the method may be especially useful in the hydrogenation of halocarbons for example chlorofluorocarbons (CFCs), and hydrohalocarbons for example hydrochlorofluorocarbons (HCFCs), especially where the chlorine is preferentially or selectively replaced by hydrogen.

Chlorofluorocarbons (CFCS) have been widely used for their excellence as aerosol propellants, refrigerants, cleaning solvents and foam blowing agents. However, it is now appreciated that CFCs have a harmful effect on the stratospheric ozone layer, with one CFC molecule being able to decompose up to 10⁵ ozone molecules, thus severely damaging the protection which the ozone layer affords the earth's surface against UV radiation. Therefore it has been internationally agreed to cease CFC production by 1996, except as chemical precursors. As a result it has become important to provide compounds which have similar technical properties to those of CFCs but which are environmentally acceptable.

For some 40% of the former demand for CFCs, the only alternatives which are practical with current technology are HCFCs and hydrofluorocarbons (HFCs), and these can best be synthesised by replacing chlorine in CFCs by hydrogen. Catalysts that have been proposed for this process include palladium chloride supported on activated carbon (JP 01,319,442 25 December 1989) or a 5% loading of palladium on activated carbon (UK Patent 1578933) or palladium supported on aluminium fluoride (European Patent Application 328127) or palladium supported on alumina (German Offen. 3917575). A problem with these catalytic systems is that the conversion yield of CFCs to HFCs is very low, typically around 5% and deactivate after a relatively short time, such as 2 hours. These palladium systems are also used to produce HFCs by removing chlorine from the alternative feedstock of chlorofluorohydrocarbons (e.g. HCFC-124, CHFClCF₃).

According to the present invention, there is provided a method of replacing halogen in halo-substituted hydrocarbon by hydrogen, comprising exposing the halo-substituted hydrocarbon to a catalyst in the presence of a source of hydrogen as set out in claims 1-12. The catalyst comprises a catalytic metal and a material which either has a greater affinity for halogen than has the catalytic metal, or stabilises the zero oxidation state of the catalytic metal, or both, so as to resist adsorption of halogen on the catalytic metal surface. The catalytic metal is palladium, rhodium, ruthenium, silver, gold or gallium, especially palladium. The said material contains metal different from the catalytic metal, such as zinc, aluminium, or gallium, and it may further contain combined oxygen, thus comprising or derived from an oxide, nitrate or organometallic compound such as a salt, e.g. carboxylate e.g. acetate, an especially effective combination being palladium and zinc (preferably as a zinc-oxgyen compound). The catalyst may be supported on an inert carrier such as alumina, preferably having a surface area of at least 50 m²/g. The method is preferably performed at a temperature of from 280 to 450°C, such as from 350 to 435°C, especially 400-425°C. At low temperatures, halo-substituted hydrocarbons tend to remain unreacted, while at excessive temperatures, they can react with the catalyst support.

Reactions especially amenable to the catalytic method set forth above are those wherein the halosubstituted carbon is a hydrochlorofluorocarbon or a chlorofluorocarbon, especially wherein chlorine rather than fluorine is replaced by hydrogen, and, if suitably carried out, the main product of the method according to the invention can indeed be a fluorocarbon or a hydrofluorocarbon, i.e. all the chlorine has been replaced by hydrogen. Most suitably, the hydrocarbon is aliphatic, e.g. an alkane, e.g. methane or ethane, and thus a typical method according to the invention would be to convert CCl₂F-CClF₂ to CH₃-CF₃ (an isomerisation does appear to occur).

The invention also extends to the catalyst as set forth in claims 13-18.

By providing the said material in the catalyst, the surface of the catalytic metal has low affinity for halogen freed from the halocarbon during hydrogenation. An important factor in the low yields obtained with previously proposed catalysts is that the active sites on the palladium surface become occupied by halogen or hydrogen halide, with consequent lessening of the catalytic effect of the palladium; the active sites are shielded from the reactants in the hydrogenation. With the catalyst of the present invention, the halogen or hydrogen halide released in the hydrogenation is attracted preferentially to the said material instead of to the catalytic metal, so the active sites on the latter remain available to the reactants for a longer time.

The components of the catalyst may be in the form of an alloy or close admixture, but other types of combination may also prove useful.

The invention will now be described by way of illustration in the following Examples.

### Example 1

The catalyst of this embodiment of the invention was prepared as follows:
1g of Degussa 'C' γ-alumina support (to give high surface area) was impregnated with 5 wt% of palladium nitrate (50 mg) and a two-fold stoichiometric amount of zinc using zinc oxide (70 mg). The resultant cake was broken up and loaded into a Pyrex reactor tube where it was calcined at 503K for 6 hours followed by reduction or partial reduction at 503K for 18 hours with a 9:1 mixture of dinitrogen and dihydrogen flowing at 100 ml min⁻¹. After this reduction, which is believed to give rise to a zinc oxide which is rich in elemental zinc especially at the surface, the reactor temperature was increased to 773K for 4 hours prior to cooling to room temperature.

1,1,2-Trichlorotrifluoroethane (CFC-113) was passed over the resulting amount of catalyst at 513K with a mass flow of 96 microlitres min⁻¹ together with a stoichiometric excess of dihydrogen, and the reactor eluant was analysed by online gas chromatographic facilities. The analysis showed the presence of 1,1,2-trifluoroethane and 1-chloro-1,2,2-trifluoroethane. The conversion from the CFC-113 was greater than 60%, and the catalyst performed at this rate of conversion over a period of 48 hours and beyond without any substantial loss of activity.

Hydrogen chloride released in the hydrogenation of the CFC-113 is adsorbed on the zinc of the catalyst to form zinc chloride, in preference to adsorption on the palladium. The high yield is obtained by virtue of the palladium remaining free of this contaminant and continuing to provide active sites for the reactants. The zinc chloride can subsequently be reduced to reconstitute the zinc metal in the catalyst.

### Examples 2-7

In all these Examples (except 6, which is a comparative example where no palladium was used), the catalyst was Pd/ZnO/γ-Al₂O₃ (or Pd/Zn acetate/γ-Al₂O₃).

Palladium nitrate hydrate was used as the precursor for the catalysts. The catalyst support was gamma alumina having a surface area of 110 m²/g. Zinc oxide or zinc acetate dihydrate was added to improve catalyst performance.

The amount of palladium nitrate hydrate shown in the Table together with 1 gram of gamma alumina and the appropriate zinc compound were weighed into an evaporating dish and dissolved in 10 ml of deionised distilled water. The solution was then dried on a heating mantel. The resulting cake was ground with an agate pestle and mortar prior to calcination.

**TABLE**

| EXAMPLE | MOLE RATIO | γ-Al₂O₃ (grams) | Pd(NO₃)₂×H₂O (grams) | ZnO/Zn(ac)₂(H₂O)₂ (grams) |
|---|---|---|---|---|
| 2 | Pd:ZnO=1:2 | 1.000 | 0.108 | 0.076 |
| 3 | Pd:ZnO=1:1 | 1.000 | 0.108 | 0.038 |
| 4 | Pd:ZnO=2:1 | 1.000 | 0.108 | 0.019 |
| 5 | Pd:ZnO=1:4 | 1.000 | 0.108 | 0.152 |
| 6 | No Pd | 1.000 | - | 0.076 |
| 7 | Zn(ac)₂ used | 1.000 | 0.108 | 0.205 |

1 gram of the impregnated catalyst was placed in a reaction tube and was first calcined at 450°C for 6 hours, under 10% hydrogen in oxygen-free nitrogen, flowing at a rate of 30 ml/min. The calcination was followed by treatment for 16 hours at 230°C under 10% hydrogen in oxgyen-free nitrogen, flowing at a rate of 30 ml/min, then recalcination at 450°C for 6 hours under 10% hydrogen in oxygen-free nitrogen, flowing at a rate of 30 ml/min. Finally the catalyst was held under 10% hydrogen in oxgyen-free nitrogen at 230°C. This treatment reduced or partially reduced the zinc compound.

Oxygen-free nitrogen was set at a rate of 13.5 ml/min flowing through a reservoir containing the CFC 113 feed (CCl₂F-CClF₂). This gave a trichlorotrifluoroethane flow rate of 43 µl/min. Hydrogen flow through the bed was set at 1.5 ml/min.

The reactor pressure was maintained at 900 torr. Runs were performed with the catalyst bed at various temperatures from 70 to 450°C. The performance of each catalyst is shown in the following tables:-

### EXAMPLE 2

| | | Of the reacted products, percentage which was: | | |
|---|---|---|---|---|
| TEMPERATURE °C | CONVERSION of feedstock CFC 113 (CCl₂FCClF₂) | HFC 143a CF₃CH₃ | HCFC 133a CF₃-CH₂Cl | OTHERS |
| 70 | 0.00 | - | - | - |
| 115 | 0.00 | - | - | - |
| 152 | 0.49 | 100.00 | - | - |
| 168 | 0.54 | 100.00 | - | - |
| 196 | 1.70 | 100.00 | - | - |
| 200 | 3.88 | 100.00 | - | - |
| 230 | 4.62 | 100.00 | - | - |
| 250 | 4.63 | 100.00 | - | - |
| 275 | 5.62 | 99.32 | 0.68 | - |
| 300 | 9.18 | 88.55 | 11.45 | - |
| 316 | 8.22 | 85.89 | 14.03 | 0.09 |
| 360 | 10.69 | 69.12 | 16.90 | 13.98 |
| 375 | 18.52 | 43.95 | 44.04 | 12.01 |
| 400 | 66.14 | 29.38 | 64.26 | 6.35 |
| 450 | 95.30 | 22.92 | 70.91 | 6.17 |

### EXAMPLE 3

| | | Of the reacted products, percentage which was: | | | |
|---|---|---|---|---|---|
| TEMPERATURE °C | CONVERSION % | HFC 143a | HCFC 133a | HCFC 123a CHClF-CClF₃ | OTHERS |
| 280 | 15.99 | 38.82 | 59.76 | 1.42 | - |
| 330 | 12.91 | 15.34 | 82.53 | 2.13 | - |
| 370 | 19.06 | 8.95 | 83.16 | 5.26 | 2.63 |
| 385 | 20.58 | 30.23 | 59.17 | 5.42 | 5.17 |
| 390 | 42.90 | 64.45 | 22.07 | 7.38 | 6.10 |
| 400 | 49.60 | 67.72 | 14.40 | 10.15 | 7.73 |

### EXAMPLE 4

| | | Of the reacted products, percentage which was: | | | |
|---|---|---|---|---|---|
| TEMPERATURE °C | CONVERSION % | HFC 143a | HCFC 133a | HCFC 123a | OTHERS |
| 230 | 7.19 | 10.83 | 18.94 | 54.14 | 16.08 |
| 280 | 8.39 | 4.81 | 17.34 | 58.04 | 19.81 |
| 330 | 10.09 | 3.22 | 11.45 | 57.21 | 28.10 |
| 380 | 19.79 | 2.67 | 2.67 | 34.66 | 60.00 |

### EXAMPLE 5

| | | Of the reacted products, percentage which was: | | |
|---|---|---|---|---|
| TEMPERATURE °C | CONVERSION % | HFC 143a | HCFC 133a | HCFC 123a |
| 280 | 61.93 | 98.41 | - | 1.59 |
| 330 | 77.14 | 98.11 | 0.55 | 1.26 |
| 380 | 83.13 | 97.00 | 1.86 | 1.14 |
| 400 | 83.85 | 93.77 | 4.42 | 1.81 |
| 420 | 94.55 | 85.14 | 13.42 | 1.43 |
| 450 | 97.96 | 93.93 | 3.18 | 2.89 |

### EXAMPLE 6 (comparative)

1 gram of the ZnO/γ-Al₂O₃ catalyst, with no palladium, was calcined and reacted as described. The catalyst bed was maintained at temperatures between 230 and 420°C. No hydrodechlorinated products were detected. A white powdery deposit was observed on the reaction tube when working at catalyst bed temperatures in the region of 230-300°C. This was suspected to be a zinc halide, possibly ZnCl₂ as its boiling point is in this region.

### EXAMPLE 7

| | Of the reacted products, percentage which was: | | |
|---|---|---|---|
| TEMPERATURE °C | CONVERSION % | HFC 143a | HCFC 133a |
| 230 | 95.27 | 58.82 | 41.18 |
| 280 | 96.92 | 73.56 | 26.44 |
| 380 | 97.83 | 77.77 | 22.23 |
| 400 | 94.21 | 84.21 | 15.79 |
| 420 | 91.83 | 91.48 | 8.52 |
| 450 | 88.28 | 92.19 | 7.81 |

1 gram of Pd/C catalyst (Aldrich 5% palladium on activated carbon) was calcined at 2300C for 2 hours and reacted as described in UK Patent 1578933. Eluant samples were taken on a half hourly basis. Initially a conversion of 4-5% was obtained; however after just 2 hours the catalyst became deactivated and the reaction ceased.

The conversion efficiency of a catalyst according to the present invention, on the other hand, showed a deactivation rate of 0.6% per hour and thus remained at at least half its original value for 20-25 hours, then falling more sharply to around 4% at 30 hours.

## Claims

1. A method of replacing halogen in halo-substituted hydrocarbon by hydrogen, comprising exposing the halo-substituted hydrocarbon to a catalyst in the presence of a source of hydrogen, the catalyst comprising palladium, rhodium, ruthenium, silver, gold or gallium, characterised in that the catalyst also comprises a material which either has a greater affinity for halogen than has the catalytic metal, or stabilises the zero oxidation state of the catalytic metal, or both, wherein the said material contains zinc, aluminium or (provided the catalyst does not comprise gallium) gallium.

2. A method according to Claim 1, wherein the said material is present in elemental form alloyed or closely admixed with said catalytic metal, and/or contains combined oxygen.

3. A method according to Claim 2, wherein said material comprises or is derived from an oxide or nitrate or organometallic compound.

4. A method according to any preceding claim, wherein the catalyst is supported on an inert carrier.

5. A method according to Claim 4, wherein the inert carrier is a ceramic.

6. A method according to Claim 5, wherein the ceramic is alumina.

7. A method according to any preceding claim, wherein the halosubstituted carbon is a hydrochlorofluorocarbon or a chlorofluorocarbon.

8. A method according to Claim 7, wherein chlorine rather than fluorine is replaced by hydrogen.

9. A method according to any preceding claim, wherein the hydrocarbon is aliphatic.

10. A method according to Claim 9, wherein the hydrocarbon is an alkane.

11. A method according to any preceding claim, when performed at a temperature of from 280 to 450°C.

12. A method according to Claim 11, when performed at a temperature of from 350 to 435°C.

13. A catalyst for use in a method according to any preceding claim, comprising a catalytic metal being palladium, rhodium. ruthenium, silver, gold or gallium, and a material having a greater affinity for halogen than does the catalytic metal, wherein the said material contains zinc, aluminium or (subject to the catalytic metal not being gallium) gallium.

14. A catalyst according to Claim 13, wherein the said material is present in elemental form alloyed or closely admixed with said catalytic metal, and/or contains combined oxygen.

15. A catalyst according to Claim 14, wherein said material comprises or is derived from an oxide or nitrate or organometallic compound.

16. A catalyst according to Claim 13, 14 or 15, wherein the catalyst is supported on an inert carrier.

17. A catalyst according to Claim 16, wherein the inert carrier is a ceramic.

18. A catalyst according to Claim 17, wherein the ceramic is alumina.

## Patentansprüche

1. Ein Verfahren zum Austausch von Halogen in halogensubstituiertem Kohlenwasserstoff durch Wasserstoff, welches beinhaltet, den halogensubstituierten Kohlenwasserstoff in Anwesenheit einer Wasserstoffquelle einem Katalysator auszusetzen, wobei der Katalysator Palladium, Rhodium, Ruthenium, Silber, Gold oder Gallium beinhaltet, dadurch gekennzeichnet, daß der Katalysator auch einen Stoff beinhaltet, der entweder eine größere Affinität zu Halogen aufweist als das katalytische Metall oder den Nulloxidationszustand des katalytischen Metalls stabilisiert oder beides, wobei der Stoff Zink, Aluminium oder (vorausgesetzt der Katalysator enthält kein Gallium) Gallium enthält.

2. Verfahren nach Anspruch 1, wobei der Stoff in elementarer Form mit dem katalytischen Metall legiert oder diesem eng zugemischt vorliegt und/oder gebundenen Sauerstoff enthält.

3. Verfahren nach Anspruch 2, wobei der Stoff ein Oxid oder Nitrat oder eine metallorganische Verbindung enthält oder von diesen abgeleitet ist.

4. Verfahren nach einem der vorhergehenden Ansprüche, wobei der Katalysator auf einem inerten Trägerstoff gehalten wird.

5. Verfahren nach Anspruch 4, wobei der inerte Trägerstoff ein Keramikstoff ist.

6. Verfahren nach Anspruch 5, wobei der Keramikstoff Tonerde ist.

7. Verfahren nach einem der vorhergehenden Ansprüche, wobei der halogensubstituierte Kohlenstoff ein Chlorfluorkohlenwasserstoff oder ein Chlorfluorkohlenstoff ist.

8. Verfahren nach Anspruch 7, wobei eher Clor als Fluor durch Wasserstoff ausgetauscht wird.

9. Verfahren nach einem der vorhergehenden Ansprüche, wobei der Kohlenwasserstoff aliphatisch ist.

10. Verfahren nach Anspruch 9, wobei der Kohlenwasserstoff ein Alkan ist.

11. Verfahren nach einem der vorhergehenden Ansprüche, bei einer Temperatur von 280 bis 450°C durchgeführt.

12. Verfahren nach Anspruch 11, bei einer Temperatur von 350 bis 435°C durchgeführt.

13. Ein Katalysator zur Verwendung in einem Verfahren gemäß einem der vorhergehenden Ansprüche, der ein katalytisches Metall, das Palladium, Rhodium, Ruthenium, Silber, Gold oder Gallium ist, und einen Stoff mit einer größeren Affinität zu Halogen als die des katalytischen Metalls enthält, wobei der Stoff Zink, Aluminium oder (falls das katalytische Metall nicht Gallium ist) Gallium enthält.

14. Katalysator nach Anspruch 13, wobei der Stoff in elementarer Form mit dem katalytischen Metall legiert oder diesem eng zugemischt vorliegt und/oder gebundenen Sauerstoff enthält.

15. Katalysator nach Anspruch 14, wobei der Stoff ein Oxid oder Nitrat oder eine metallorganische Verbindung enthält oder von diesen abgeleitet ist.

16. Katalysator nach Ansprüchen 13, 14 oder 15, wobei der Katalysator auf einem inerten Trägerstoff gehalten wird.

17. Katalysator nach Anspruch 16, wobei der inerte Trägerstoff ein Keramikstoff ist.

18. Katalysator nach Anspruch 17, wobei der Keramikstoff Tonerde ist.

## Revendications

1. Un procédé pour remplacer un halogène dans un hydrocarbure halogéné par de l'hydrogène, comprenant l'exposition de l'hydrocarbure halogéné à un catalyseur en présence d'une source d'hydrogène, le catalyseur comprenant du palladium, du rhodium, du ruthénium, de l'argent, de l'or ou du gallium, caractérisé en ce que le catalyseur comprend aussi une matière qui soit a une plus grande affinité pour l'halogène que le métal catalytique, soit stabilise l'état d'oxydation à zéro du métal catalytique, soit les deux, dans lequel ladite matière contient du zinc, de l'aluminium ou (à condition que le catalyseur ne comprenne pas de gallium) du gallium.

2. Un procédé selon la revendication 1, dans lequel ladite matière est présente sous forme élémentaire alliée ou étroitement mélangée avec ledit métal catalytique, et/ou contient de l'oxygène combiné.

3. Un procédé selon la revendication 2, dans lequel ladite matière comprend ou provient d'un composé d'oxyde ou de nitrate ou organométallique.

4. Un procédé selon l'une quelconque des revendications précédentes, dans lequel le catalyseur est soutenu par un vecteur inerte.

5. Un procédé selon la revendication 4, dans lequel le vecteur inerte est une céramique.

6. Un procédé selon la revendication 5, dans lequel la céramique est l'alumine.

7. Un procédé selon l'une quelconque des revendications précédentes, dans lequel le carbone halogéné est un hydrochlorofluorocarbure ou un chlorofluorocarbone.

8. Un procédé selon la revendication 7, dans lequel du chlore plutôt que du fluor est remplacé par de l'hydrogène.

9. Un procédé selon l'une quelconque des revendications précédentes, dans lequel l'hydrocarbure est aliphatique.

10. Un procédé selon la revendication 9, dans lequel l'hydrocarbure est un alcane.

11. Un procédé selon l'une quelconque des revendications précédentes, lorsqu'il est réalisé à une température allant de 280 à 450°C.

12. Un procédé selon la revendication 11, lorsqu'il est réalisé à une température allant de 350 à 435°C.

13. Un catalyseur à utiliser dans un procédé selon l'une quelconque des revendications précédentes, comprenant un métal catalytique étant du palladium, du rhodium, du ruthénium, de l'argent, de l'or ou du gallium, et une matière ayant une plus grande affinité pour l'halogène que le métal catalytique, dans lequel ladite matière contient du zinc, de l'aluminium ou (à condition que le métal catalytique ne soit pas du gallium) du gallium.

14. Un catalyseur selon la revendication 13, dans lequel ladite matière est présente sous forme élémentaire alliée ou étroitement mélangée avec ledit métal catalytique, et/ou contient de l'oxygène combiné.

15. Un catalyseur selon la revendication 14, dans lequel ladite matière comprend ou provient d'un composé d'oxyde ou de nitrate ou organométallique.

16. Un catalyseur selon la revendication 13, 14, ou 15, dans lequel le catalyseur est soutenu par un vecteur inerte.

17. Un catalyseur selon la revendication 16, dans lequel le vecteur inerte est une céramique.

18. Un catalyseur selon la revendication 17, dans lequel la céramique est l'alumine.
